(19) Europäisches Patentamt
European Patent Office
Office européen des brevets

(11) Publication number: 0 075 982
B1

(12) # EUROPEAN PATENT SPECIFICATION

(45) Date of publication of patent specification: 21.11.85

(51) Int. Cl.[4]: G 01 N 33/533, C 09 K 11/07

(21) Application number: 82201143.3

(22) Date of filing: 15.09.82

(54) **Immunofluorescence reagents, and the method for their preparation.**

(30) Priority: 25.09.81 IT 2416181

(43) Date of publication of application:
06.04.83 Bulletin 83/14

(45) Publication of the grant of the patent:
21.11.85 Bulletin 85/47

(84) Designated Contracting States:
AT BE CH DE FR GB LI LU NL SE

(58) References cited:
DE-A-2 557 419
DE-A-2 641 713
DE-A-2 824 917
DE-A-2 834 743
GB-A-2 021 262
GB-A-2 026 690
US-A-4 104 029

ANGEWANDTE CHEMIE; Band 89, 1977 Weinheim Y. KANAOKA "Organische Fluoreszenzreagentien für die Untersuchung von Enzymen und Proteinen" pages 142 to 152

(73) Proprietor: ANIC S.p.A.
Via Ruggero Settimo, 55
I-90139 Palermo (IT)

(72) Inventor: Baroncelli, Vittorio
Via Tagliamento, 20
I-00198 Roma (IT)
Inventor: Colapicchioni, Claudio
Via di San Castulo, 3
I-00182 Roma (IT)
Inventor: Giannini, Ivo
Via Aurelia, 1100
I-00166 Roma (IT)
Inventor: Porcelli, Filippo
Via del Forte Braschi, 87
I-00167 Roma (IT)

(74) Representative: Roggero, Sergio et al
Ing. Barzanò & Zanardo Milano S.p.A. Via Borgonuovo 10
I-20121 Milano (IT)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 075 982 B1

Courier Press, Leamington Spa, England.

**Description**

In the clinical diagnostics, the quantitative determination, by immunological methods, of small amounts of compounds such as hormones, drugs or metabolites, is extremely important. One of the methods for quantifying immunoreagents is to mark the antigens and antibodies with fluorophores such as fluorescein or rhodamine isothiocyanate. The main problem connected with the use of such method is the inherent fluorescence of the biological samples. In this respect, many substances, such as flavins, pyridine coenzymes and serum proteins fluoresce intensely in those regions of the spectrum in which also the fluorophores which are normally used as probes exhibit fluorescence. The use of probes which are fluorescent in the red-near infrared region (600—900 nm) and thus fairly distant from the inherent fluorescence of the samples being tested (300—450 nm), would enable the background due to natural substance to be eliminated, the sensitivity of the method being thus improved. Many dyes which are free in solution are known to have these spectral properties, even though the intensity of their fluorescence is fairly weak, but what is by no way obvious is that they retain these properties if their structure is somehow modified by a covalent bond with another molecule.

An improvement has now been achieved, and is the subject matter of this invention, by covalently bonding dyes which fluroesce in the red-near infrared region to various proteins, by using bifunctional reagents which react with $—NH_2$ groups of the fluorophores, without such dyes losing their spectral characteristics and facturally improving them by increasing their fluorescence by as much as 2 to 4 times, as can be appreciated when observing the accompanying drawings.

In the drawings:

Figure 1 shows the emission spectra of an anti-gammaimmunoglobulinantibody-Nile blue A conjugate (curve 2) and of a Nile blue A solution (curve 1), respectively.

The fluorophore concentration is as low as $2 \times 10^{-6}$M, and the excitation wavelength is 600 nm. These spectra had been obtained with a PERKIN-ELMER MPF—2A spectrofluorometer fitted with a HAMAMATSU R446 photomultiplier. In the plots, the abscissa is the wavelength, in nm, and the ordinate is the fluroescence, plotted in arbitrary units.

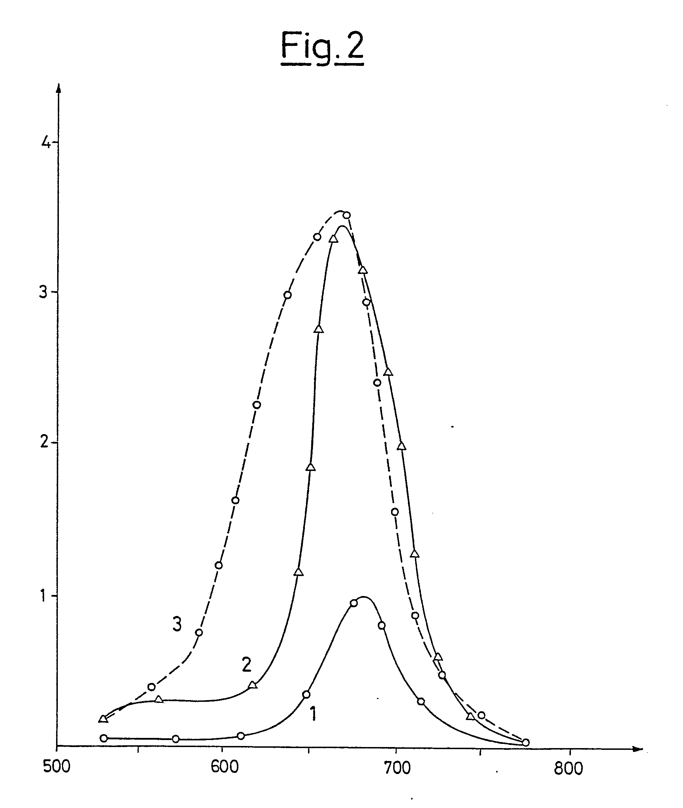

Figure 2 shows the emission spectra of the Protein A-Nile blue A conjugate (curve 3) and of the HCS-Nile blue A (curve 2) conjugate, respectively, and curve 1 shows the spectrum of the free Nile blue A, all obtained by excitation at $\lambda = 515$ nm with an Argon laser. The fluorophore concentration is $6 \times 10^{-6}$M, and the abscissa is the wavelength in nm, and the ordinate is the fluorescence plotted in arbitrary units again.

As a general rule, the bifunctional reagent, as represented by the cross-linking agent, reacts directly both with the protein and with the dye, to form complexes having the general formula

$$[\text{Dye}\,]_{\overline{n}}\ \text{L}_{\overline{m}}\ \text{Protein}$$

If the agent is a carbo-diimide, than, since these reagents act by activating the carboxyl groups and the amino group of the dye, complexes are obtained, having the general formula:

$$\begin{array}{c}\text{Dye—}\underset{\displaystyle \text{H}}{\underset{|}{\text{N}}}\text{—}\underset{\displaystyle \text{O}}{\underset{\|}{\text{C}}}\text{—Protein}\end{array}$$

The protein moiety of these conjugates also retains its biological properties, such as the immunological ones, if it is an antigen or an antibody. It should be noted that the use of these immunofluorescent reagents does not cause any instrumental difficulties because all that is necessary is to connect an infrared-sensitive photomultiplier to a conventional spectrofluorometer. Particularly sensitive measurements can be taken by exciting the fluorophore involved with a continuous, or a pulsed laser radiation of red light or near-infrared, such as a helium-laser radiation, a ruby-laser radiation, or, more easily, a dye-laser radiation.

The use of the conjugates referred to above, more particularly fluorescent antibodies, in microscopy, is by no means difficult, because an infrared sensitive film is all that is required. It becomes also possible, with advantage, to discriminate, in the same microscopic preparation, zones stained with antibodies which have been marked with conventional fluorophores, which fluoresce in the green-yellow region of the spectrum.

The present invention, therefore, relates to novel immunofluorescent reagents having the general formula:

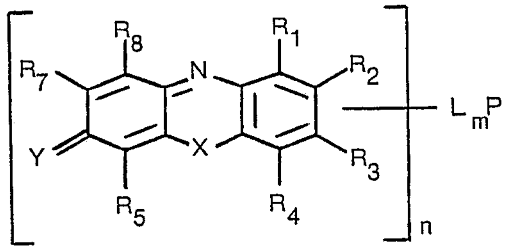

wherein:
*n* is an integer from 1 to 4;
*m* is an integer equal to *n*, unless the cross-linking agent is a carbo-diimide: if so, m is zero;
P is a protein;
L is a cross-linking agent residue;
X is an oxygen atom or a sulphur atom;
Y is a member selected from NH, $\overset{\oplus}{NH_2}$, NR′ and $\overset{\oplus}{NR'R''}$,

wherein R′ and R″, which may be the same or different, are optionally substituted alkyl, aryl, aralkyl or alkylaryl radicals, or a OCOR′ grop wherein R′ is as defined above; $R_1$, $R_2$, $R_3$, $R_4$, $R_5$, $R_7$ and $R_8$, which may be the same or different, are selected from hydrogen atoms, amino groups and optionally substituted alkyl, aryl, alkaryl or aralkyl radicals, and $R_1$ and $R_2$ taken together and/or $R_7$ and $R_8$ taken together can also represent a bivelent radical which forms a condensed ring structure with the aromatic nucleus.

At least one of the $R_1 \ldots R_8$ groups, most frequently the $R_3$, must be an amino group which, when reacted with the cross-linking agent concerned, gives rise to an —NH—L, or an —N=L group. The reagents in question are prepared by reacting together a protein, P, a cross-linking agent, L, and a dye, the dye being a member selected from phenoxazines and phenothiazines having an absorption in the visible range with a $\lambda_{max}$ between 550 and 800 nm, and having at least one free —$NH_2$ group and a fluorescence with emission between 600 and 900 nm, the cross-linking agent being a member selected from bifunctional reagents containing functional groups capable of reacting with the amino group of the dye concerned.

A few examples of the compounds which can be used for preparing the reagents in question will be enumerated hereinafter by way of example only.

Proteins

1) Protein A from *Staphilococcus aureus*
2) Protein hormones
3) Gamma-immunoglobulins (Ig) of the various classes such as IgG, IgM, IgA, IgD and IgE.

Dyes

1) NILE BLUE A (C.I. 51180)

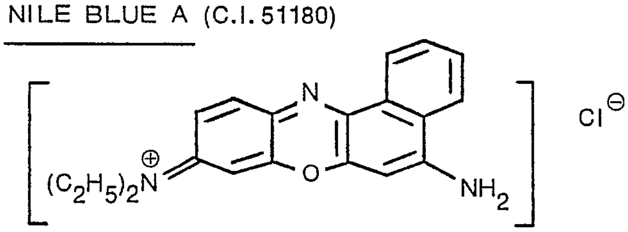

2) CRESYL VIOLET

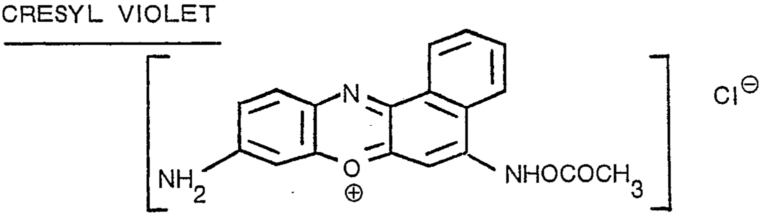

3) TOLUIDINE BLUE (C.I. 52840)

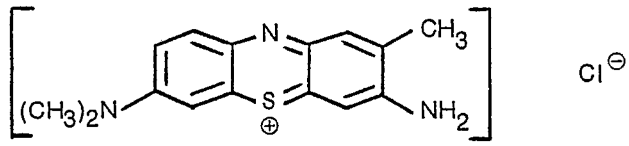

4) BRILLIANT CRESYL BLUE (C.I. 51010)

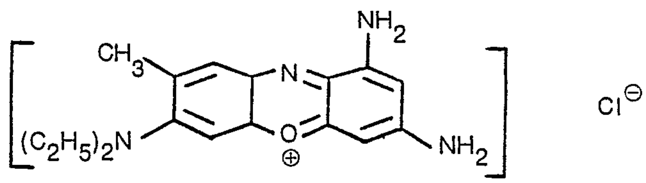

Cross-linking agents

1) a diisocyanate ahving the general formula O=C=N—R—N=C=O, wherein R is an optionally substituted alkylene, arylene, alkarylene or aralkylene radical;

2) a carbo-diimide having the general formula R—N=C=N—R', wherein R and R', which are the same or different, are optionally substituted alkyl, aryl, alkaryl or aralkyl radicals;

3) a dialdehyde having the general formula CHO—R—CHO wherein R is an optionally substituted alkylene, arylene, alkarylene or aralkylene;

4) an ethylene-maleic acid anhydride copolymer (EMA) having the general formula:

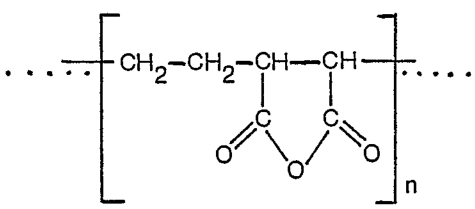

5) a dinitrobenzene having the general formula:

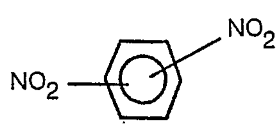

The reaction between the protein, the cross-linking agent and the dye can either be carried out starting from the substrates alone, or can be carried out in the presence of a medium compatible with the compounds concerned, such as water, an aqueous solution of one or more salts, or a buffered liquid. The pH of the medium may range from 4,5 to 9,5: moreover, small amounts of organic solvents may be added to increase the dyestuff solubility. The reaction temperature may generally range from 4°C to the protein denaturation temperature: more particularly it is in the ambient temperature range.

A large excess of the dye over the protein is generally advisable to prevent interprotein reactions: the excess is from 4 to 50 times in terms of molar concentration.

Likewise, a large excess of the cross-linking agent over the total quantity of protein plus dye is recommendable for a smooth reaction run.

The invention will be better understood from the ensuring non-limiting partical examples of its application.

Example 1

The immunoglobulin fraction of an anti human immunoglobulin G rabbit serum (anti h IgG) was marked both with Nile blue A (CI 51180) and with toluidine blue (CI 52040), using glutaraldehyde as the bifunctional reagent.

Glutaraldehyde is added to a solution containing 2 mg of anti h IgG and $1.3 \times 10^{-4}$ mmoles of Nile blue A or toluidine blue in 1 ml of potassium phosphate buffer (0.01 M pH 7.1) until the final glutaraldehyde concentration is 0.05%.

The reaction is allowed to proceed for 30 minutes under stirring at ambient temperature, and is then blocked by trapping the free aldehyde groups with sodium bisulphite. The unreacted dye is eliminated by chromatography over Sephadex® G25.

In a typical experiment, a fluorescent anti h IgG — Nile blue A conjugate is obtained with a molar protein: dye ratio of 1 : 1.8, or an anti h IgG — toluidine blue conjugate with a molar ratio of 1 : 1.2.

The dye concentration was calculated using for the Nile blue A a molar extinction coefficient of $5.8 \times 10^4$ at $\lambda = 635$ nm, and for the toluidine blue a molar extinction coefficient of $3.1 \times 10^4$ at $\lambda = 620$ nm.

The protein concentration was calulated using a $\varepsilon^{\%} = 14$ at $\lambda = 280$ nm, and correcting for dye absorption in the ultraviolet, or by determining the nitrogen with the Kjeldahl micromethod.

Both the conjugates significantly maintain the antibody property of binding human immunoglobulins.

Example 2

A conjugate between human chorionic somatomammotropin (HCS) and a dye of the oxazine series, namely brilliant cresyl blue (CI 51010), is prepared using glutaraldehyde as the reagent. Glutaraldehyde is added to 1 ml of sodium phosphate buffer at pH 7.8, 0.05 M containing 2.5 mg of HCS and $5 \times 10^{-4}$ mmoles of dye, until the final glutaraldehyde concentration is 0.1%. After 1 hour of reaction at ambient temperature, the solution is treated as in example 1 to obtain a conjugate with a molar HCS : brilliant cresyl blue ratio of 1 : 1.

The ratio was calulated using for the dye a molar extinction coefficient of 16500 at $\lambda = 633$, and for the HCS a $\varepsilon^{\%} = 8.3$ at $\lambda = 278$.

Example 3

Using glutaraldehyde as the cross-linking agent, then under the same experimental conditions as example 1 a conjugate is prepared between Nile blue A and Protein A from *Staphylocccus aureus.*

The molar protein-dye ratio of this conjugate is 1 : 1.5. In this case, when not using the Kjeldahl micromethod, a $\varepsilon^{\%} = 1.65$ at $\lambda = 275$ was used for determining the Protein A.

Example 4

Nile blue A (CI 51180) is used for marking anti human immunoglobulin (anti h IgG), using as the bifunctional reagent toluylene-2,4-diisocyantate (TDIC), of which the structural formula is

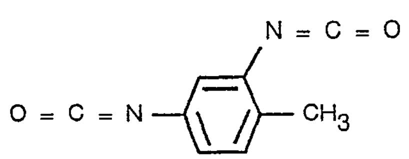

The reaction proceeds in two steps. In the first, the isocyanate group in position 4, which is less sterically committed, reacts with the $NH_2$ of the anti hIgG.

In the second step, Nile blue A is added at 37°C to the anti hIgG—TDIC conjugate containing no free TDIC, so that the second isocyanate group reacts with the amino group of the dye.

Step 1—

0.05 ml of TDIC are added to a solution of 5 mg of γ-immunoglobulin in potassium phosphate buffer of pH 7.5, 0.05 M, cooled to 0°C. The reaction is allowed to proceed for about 30 minutes at 0°C under vigorous stirring, the mixture then being centrifuged to remove the unreacted diisocyanate which precipitates in aqueous solutions at 0°C. The supernatant is allowed to incubate again at 0°C for a further hour, in order to enable any dissolved TDIC to react.

Step 2—

0.1 ml of phosphate buffer containing $10^{-4}$ mmoles of Nile blue A are added to the solution of step 1. The resultant solution is kept at 37°C for 1 hour under stirring, and is then dialysed against 0.1 M ammonium carbonate in order to destroy any unreacted isocyanate group. The free dye is removed by dialysis against distilled water or neutral buffer followed by chromatography over Sephadex® G25.

The resultant conjugate has a molar protein: Nile blue A ratio of 1:2.4, calculated using the methods and extinction coefficients for protein and dye given in example 1.

The fluorescence intensity of this conjugate (see Figure 1) is much higher than that obtained on exciting the free chromophore at the same concentration.

Example 5

Nile blue A (CI. 51180) is conjugated with Protein A from *Staphylococcus aureus* using 1-ethyl-3-(3-dimethylaminopropyl)-carbodiimide (ECDI), of which the structural formula is $CH_3—CH_2—N=C=N—CH_2—CH_2—CH_2N—(CH_3)_2$.

50 mg of ECDI are added toa solution containing 5 mg of Protein A and an excess of Nile bluye A to the extent of about ten times molar in 1.2 ml of distilled water. The reaction is allowed to proceed under stirring for 7 hours at ambient temperature, while maintaining the pH between 6 and 6.8 by adding dilute HCl, after which the product is dialysed against 4—5 water changes over 48 hours. The colloidal suspension which can form in the dialysis bag is eliminated by centrifuging, and any free dye still in solution is removed by chromatography over Sephadex® G25.

The Nile blue A concentration in the conjugate is determined using a molar extinction coefficient in water of $5.8 \times 10^4$ at $\lambda = 635$. The protein concentration is calculated using a $\varepsilon^{\%} = 1.65$ $\lambda = 275$ nm and correcting for the dye absorption in the ultraviolet or, in a more precise manner, by determining the nitrogen with the Kjeldahl micromethod.

In a typical reaction, a conjugate is obtained with a molar Protein A: Neil blue A ratio of $1{:}1 \equiv 1.5$ (this radio can be varied by varying the reaction time and temperature) and a fluorescence intensity at $\lambda = 680$ nm which is greater than that obtained by exciting the free chromophore at the same concentration. The Protein A marked in this manner maintains its property of binding immunoglobulins, as demonstrated by the passive hemoagglutination test, comprising agglutination of ram erythrocytes sensitised with rabbit G immunoglobulins by Protein A — Nile blue A.

Example 6

A protein hormone, namely human chorionic somatomammotropin (HCS) is marked with Nile blue A in the presence of ECDI using the procedure illustrated in example 5, to which reference should be made for details.

The reagent concentrations are 2.5 mg of HCS, $10^{-3}$ mmoles of Nile blue A dissolved in 0.05 ml of ethanol, and 45 mg of ECDI, all in 2 ml of water.

The protein concentration is determined using a $\varepsilon^{\%} = 8.3$ at $\lambda = 278$.

The fluorescent conjugate obtained has a molar HCS: dye ratio of 1:1, and the hormone maintains its antigen properties when incubated with an anti HCS antiserum.

Example 7

Toluidine blue (CI 52040) is used to mark rabbit γ-immunoglobulins using ECDI.

The reaction conditions are as follows.

The reaction mixture, consisting of 3 mg of γ-immunoglobulins and $2 \times 10^{-4}$ mmoles of toluidine blue in 2 ml of water is adjusted to pH 5.6 by dilute HCl. 25 mg of ECDI are added, and the reaction is allowed to proceed under stirring at ambient temperature. After about 2 hours, a further 25 mg of ECDI are added, and the reaction is allowed to proceed for a further 2 hours.

The solution is finally dialysed against water or a neutral buffer of low ionic force for 48—72 hours with various changes. Using a molar extinction coefficient of $3.1 \times 10^4$ for the dye at $\lambda = 620$ nm, and a $\varepsilon^{\%} = 14$ for the protein at $\lambda = 280$ nm, a molar protein: toluidine blue ratio of 1:1.1 is calculated for the prepared conjugate.

The γ-immunoglobulin marked in this manner maintains its immunological properties in the presence of anti γ-immunoglobulin.

**Claims**

1. Immunofluorescence reagents of general formula

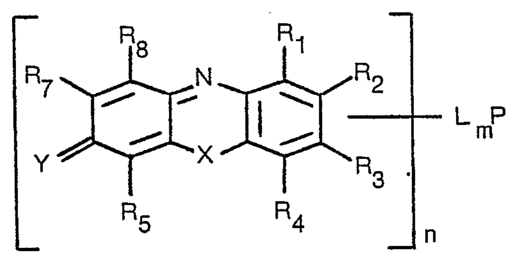

in which

n is an integer from 1 to 4;

m is an integer equal to n, unless the cross-linking agent is a carbo-diimide; if so m is zero;

P is a protein;

L is the residue of a cross-linking agent;

X is an oxygen or sulphur atom;

Y is a group chosen from NH, $\overset{\oplus}{N}H_2$, NR' and $\overset{\oplus}{N}R'R''$, where R' and R'', which can be the same or different, are optionally substituted alkyl, aryl, arylalkyl or alkylaryl radicals, or a OCOR' group where R' has the aforesaid meaning;

$R_1$, $R_2$, $R_3$, $R_4$, $R_5$, $R_7$ and $R_8$, which can be the same or different, are hydrogen atoms or amino groups or optionally substituted alkyl, aryl, alkylaryl or arylalkyl radicals;

$R_1$ and $R_2$ taken together and/or $R_7$ and $R_8$ taken together can also be bivalent radicals which form a condensed ring with the nucleus;

at least one of $R_1$ to $R_8$ (most frequently $R_3$) must be an amino group which on reacting with the cross-linking reagent gives rise to a —NH—L or —N = L— group.

2. A method for preparing the reagents defined in Claim 1, characterized in that a protein, P, a cross-linking agent, L, and a dye are reacted together, the dye being selected from phenoxazines and phenothiazines, having an absorption in the visible range with a $\lambda_{max}$ between 550 and 800 nm, and having at least one free —$NH_2$ group and a fluorescence with emission between 600 and 900 nm, the crosslinking agent being selected from bifunctional reagents containing functional groups capable of reacting with the amino group of the dye concerned.

3. Method according to Claim 2, characterized in that the reaction is carried out either starting from the substrates as such, or in the presence of a medium compatible with the reactants.

4. A method according to Claim 3, characterized in that the reaction is carried out in the presence of water, or an aqueous solution of one or more salts, or a buffered liquid.

5. A method according to Claim 2, characterized in that the reaction occurs at a pH between 4,5 and 9,5.

6. A method according to Claim 2, characterized in that the reaction temperature ranges from 4°C to the denaturation temperature of the protein concerned.

7. A method according to Claim 2, characterized in that the reaction is carried out with a large excess of the dye over the protein.

8. A method according to Claim 7, characterized in that the reaction is carried out with an amount of the dye, expressed in molar terms, which varies from 4 to 50 times that of the protein.

9. A method according to Claim 2, characterized in that the reaction is carried out with an excess of the bifunctional cross-linking agent over the total quantity of the protein plus the dye.

**Patentansprüche**

1. Immunofluoreszenz-Reagentien der allgemeinen Formel

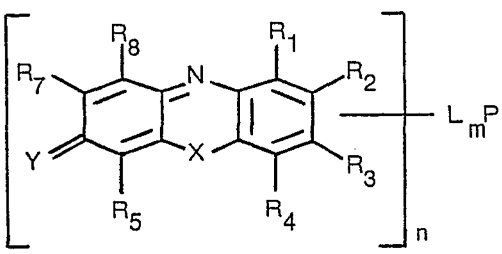

in der

n eine ganze Zahl von 1 bis 4 ist,

m eine ganze Zahl gleich n ist, soweit das Vernetzungsmittel kein Carbodiimid ist, falls dies der Fall ist, ist m gleich 0,

P ein Protein ist,

L der Rest eines Vernetzungsmittels ist,

X ein Sauerstoff-oder Schwefelatom bedeutet,

Y eine Gruppe bedeutet, ausgewählt aus NH, $\overset{\oplus}{N}H_2$, NR und $\overset{\oplus}{N}R'R''$, wobei R' und R'', die gleich oder verschieden sein können, gegebenenfalls substituierte Alkyl-, Aryl-, Arylalkyl- oder Alkylarylreste bedeuten, oder eine OCOR'—Gruppe, wobei R' die oben angegebene Bedeutung hat, und

$R_1$, $R_2$, $R_3$, $R_4$, $R_5$, $R_7$ und $R_8$, die gleich oder verschieden sein können, Wasserstoffatome, oder Aminogruppen oder gegebenenfalls substituierte Alkyl-, Aryl-, Alkylaryl- oder Arylalkylreste bedeuten, $R_1$ und $R_2$ zusammengenommen und/oder $R_7$ und $R_8$ zusammengenommen auch zweiwertige Reste bedeuten können, die einen kondensierten Ring mit dem aromatischen Kern bilden,

zumindest einer der Reste $R_1$ bis $R_8$ (am häufigsten $R_3$) eine Aminogruppe sein muß, die bei Umsetzung mit dem Vernetzungsmittel zur Bildung einer —NH—L oder —N = L—Gruppe führt.

2. Verfahren zur Herstellung der in Anspruch 1 angegebene Reagentien, dadurch gekennzeichnet, daß ein Protein P, ein Vernetzungsmittel L und ein Farbstoff miteinander umgesetzt werden, wobei der Farbstoff ausgewählt wird aus Phenoxazinen und Phenothiazinen mit einer Absorption im sichtbaren Bereich mit $\lambda_{max}$ zwischen 550 und 800 nm, und der zumindest eine freie-$NH_2$-Gruppe besitzt und eine Fluoreszenz-Emission zwischen 600 und 900 nm und wobei das Vernetzungsmittel ausgewählt ist aus bifunktionellen Reagentien, enthaltend funktionelle Gruppen, die imstande sind, mit der Aminogruppe des betreffenden Farbstoffs zu reagieren.

3. Verfahren nach Anspruch 2, dadurch gekennzeichnet, daß die Reaktion durchgeführt wird entweder ausgehend von den Substraten als solchen oder in Gegenwart eines Mediums, das mit den Reaktionspartnern verträglich ist.

4. Verfahren nach Anspruch 3, dadurch gekennzeichnet, daß die Reaktion in Gegenwart von Wasser oder einer wäßrigen Lösung eines oder mehrerer Salze oder einer gepufferten Flüssigkeit durchgeführt wird.

5. Verfahren nach Anspruch 2, dadurch gekennzeichnet, daß die Reaktion bei einem pH-Wert zwischen 4,5 und 9,5 eintritt.

6. Verfahren nach Anspruch 2, dadurch gekennzeichnet, daß die Reaktionstemperatur im Bereich von 4°C bis zur Denaturierungstemperatur des betreffenden Proteins liegt.

7. Verfahren nach Anspruch 2, dadurch gekennzeichnet, daß die Reaktion mit einem großen Überschuß an Farbstoff gegenüber dem Protein durchgeführt wird.

8. Verfahren nach Anspruch 7, dadurch gekennzeichnet, daß die Reaktion mit einer Menge an Farbstoff, angegeben in molaren Werten, durchgeführt wird, die von dem 4- bis 50-fachen des Proteins liegt.

9. Verfahren nach Anspruch 2, dadurch gekennzeichnet, daß die Reaktion mit einem Überschuß an dem bifunktionellen Vernetzungsmittel gegenüber der Gesamtmenge an Protein plus Farbstoff durchgeführt wird.

**Revendications**

1. Réactifs d'immunofluorescence ayant la formule générale:

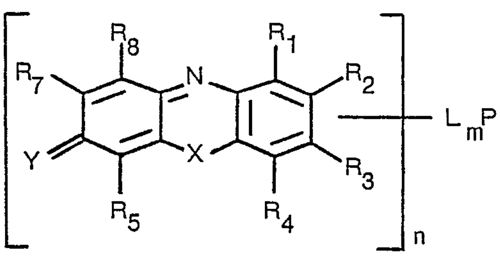

dans laquelle

n est un nombre entier de 1 à 4;

m est un nombre entier égal à n, sauf si l'agent de réticulation étant un carbo-diimide, m vaut zéro;

P représente une protéine;

L est le reste d'un agent de réticulation;

X est un atome d'oxygène ou de soufre;

Y est un groupe choisi parmi NH, $\overset{\oplus}{N}H_2$, NR' et $\overset{\oplus}{N}R'R''$, où R' et R'', qui peuvent être identiques ou différents, sont des radicaux alkyle, aryle, arylalkyle ou alkylaryle éventuellement subtitués, ou bien un groupe OCOR' dans lequel R' a les significations précédentes;

$R_1$, $R_2$, $R_3$, $R_4$, $R_5$, $R_7$ et $R_8$, qui peuvent être identiques ou différents, sont des atomes d'hydrogène ou des groupes amino ou bien des radicaux alkyle, aryle, alkylaryle ou arylalkyle éventuellement substitués;

$R_1$ et $R_2$ pris ensemble et/ou $R_7$ et $R_8$ pris ensemble peuvent être également des radicaux bivalents qui forment un noyau condensé avec le noyau aromatique;

au moins un des $R_1$ à $R_8$ (le plus fréquemment $R_3$) doit être un groupe amino qui, par réaction avec l'agent réticulant, donne naissance à un groupe —NH—L ou —N = L—.

2. Procédé pour la préparation des réactifs définis dans la revendication 1, caractérisé par le fait qu'une protéine P, un agent réticulant L, et un colorant sont mis à réagir ensemble, le colorant étant choisi parmi les phénoxazines et les phénothiazines ayant une absorption dans le domaine du spectre visible à $\lambda_{max}$ compris entre 550 et 800 nm, et ayant au moins un groupe —$NH_2$ libre et une fluorescence avec une émission comprise entre 600 et 900 nm, l'agent de réticulation étant choisi parmi les réactifs bifonctionnels contenant des groupes fonctionnels pouvant réagir avec le groupe amino du colorant en question.

3. Procédé selon la revendication 2, caractérisé par le fait que la réaction est effectuée soit en partant des substrats tels quels, soit en présence d'un milieu compatible avec les réactifs.

4. Procédé selon la revendication 3, caractérisé par le fait que la réaction est effectuée en présence d'eau ou d'une solution aqueuse d'un ou plusieurs sels, ou d'un liquide tamponné.

5. Procédé selon la revendication 2, caractérisé par le fait que la réaction a lieu à un pH compris entre 4,5 et 9,5.

6. Procédé selon la revendication 2, caractérisé par le fait que la température de réaction va depuis 4°C jusqu'à la température de dénaturation de la protéine concernée.

7. Procédé selon la revendication 2, caractérisé par le fait que la réaction est effectuée avec un grand excès du colorant par rapport à la protéine.

8. Procédé selon la revendication 7, caractérisé par le fait que la réaction est effectuée avec une quantité de colorant, exprimée en terme molaire, qui varie de 4 à 50 fois celle de la protéine.

9. Procédé selon la revendication 2, caractérisé par le fait que la réaction est effectuée avec un excès de l'agent réticulant bifonctionnel par rapport à la quantité totale de la protéine plus de colorant.

Fig.1

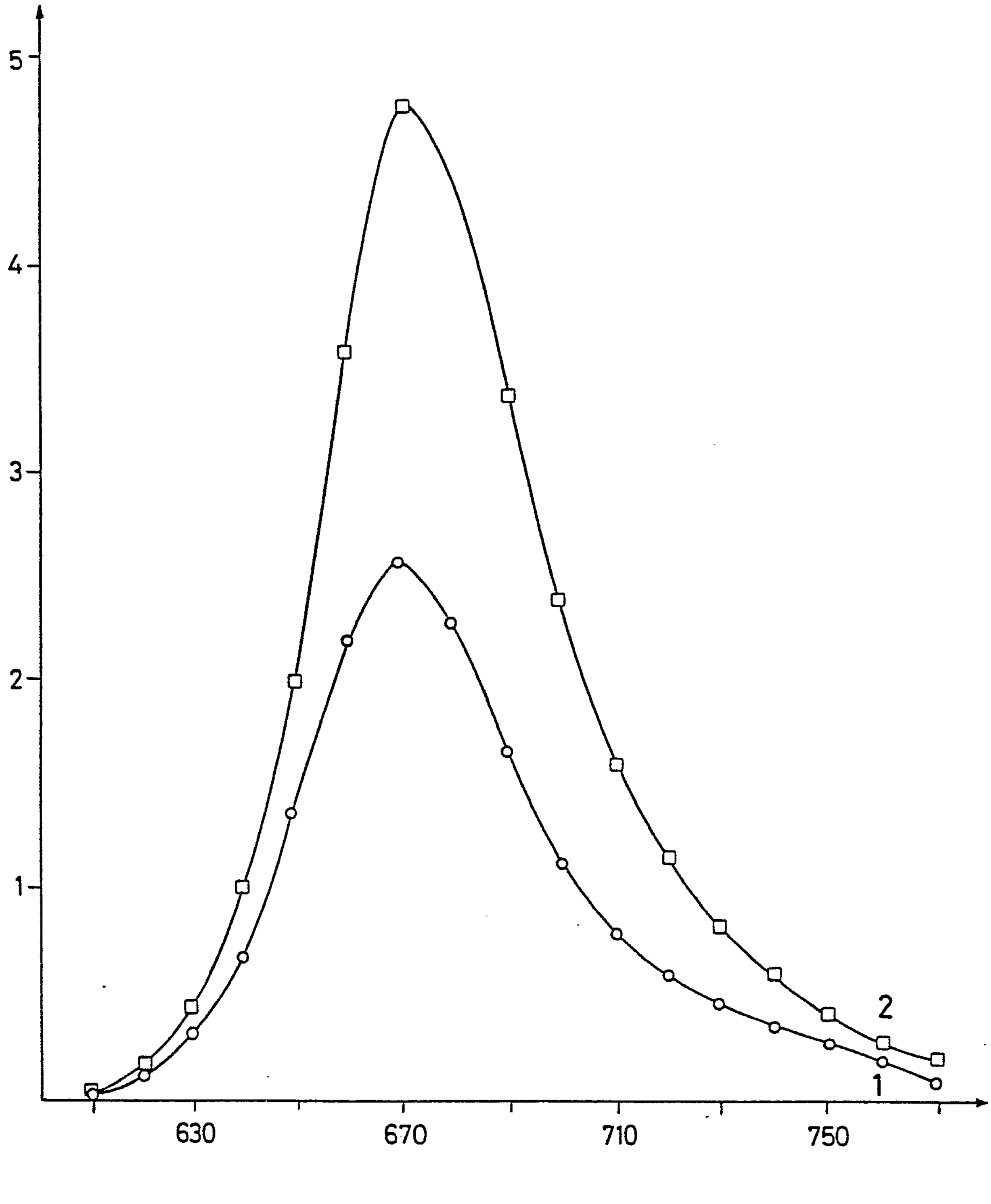

Fig. 2